# EUROPEAN PATENT APPLICATION

(11) **EP 4 715 805 A1**
(43) Date of publication of application: **25.03.2026**
(21) Application number: 24201998.2
(22) Date of filing: 23.09.2024
(51) Int. Cl.: G10K 11/35, A61B 8/00

(54) **REORIENTABLE ULTRASOUND SYSTEM**

(71) Applicant: Nederlandse Organisatie voor toegepast- natuurwetenschappelijk onderzoek TNO, 2595 DA 's-Gravenhage (NL)
(72) Inventor: KAWASAKI, Shinnosuke, 2595 DA 's-Gravenhage (NL); BAKKER, Maarten Herman, 2595 DA 's-Gravenhage (NL)
(74) Representative: V.O.

(57) **Abstract**

An ultrasound system (100) comprising a housing (10) with a contact surface (10c) for contacting an object (O), and a dynamic platform (11) comprising an ultrasound transducer (13). The dynamic platform (11) is suspended inside the housing (10) and connected to the housing (10) via a flexible connection structure (12) configured to allow reorientation of the dynamic platform (11) inside the housing (10) with respect to the contact surface (10c) for modifying a field of view (F) of the ultrasound transducer (13) on the object (O). A method of measuring an object (O), the method comprising reorienting a dynamic platform (11) inside a housing (10) with respect to a contact surface (10c) of the housing (10), while the dynamic platform (11) is held suspended via a flexible connection structure (12), for modifying a field of view (F) of the ultrasound transducer (13) on the object (O).

## Description

### TECHNICAL FIELD AND BACKGROUND

The present disclosure relates to an ultrasound system, in particular a reorientable ultrasound system and method thereof.

Operating handheld ultrasound transducers involves coordinating both rotational and tilting movements, which can be challenging and typically requires skilled professionals. As ultrasound technology becomes more widespread, there's a need for solutions that allow less experienced operators to use handheld scanners effectively, maintaining full control over rotation and tilt. This would help address the shortage of skilled operators and enable highly trained professionals to focus on other important tasks during scanning.

The limitations faced with handheld transducers are also present in ultrasound patches. These patches, comprising one or more transducer elements attached to the body with straps, belts, or adhesives, have a fixed field of view and cannot observe different directions once placed. While adding more transducer elements to the patches might seem like a solution, it would increase hardware complexity.

A new approach is needed to enhance maneuvrability of both the handheld ultrasound transducers and ultrasound patches, improving their functionality without complicating the hardware and/or ease of use.

### SUMMARY

Aspects of the present disclosure relate to an ultrasound system comprising a housing comprising a contact surface for contacting an object, and a dynamic platform comprising an ultrasound transducer. The dynamic platform is suspended inside the housing and connected to the housing via a flexible connection structure configured to allow reorientation of the dynamic platform inside the housing with respect to the contact surface for modifying a field of view of the ultrasound transducer on the object.

By disposing electrical connections to the ultrasound transducer in and/or on the flexible connection structure connecting the housing to the dynamic platform, less slack in the electrical connections disposed on the flexible connection structure is needed as compared to a bulk cable connecting to the backside of the dynamic platform.

By providing the flexible connection structure with a plurality of flexible legs, stress in the flexible connection structure caused by forces induced during reorientation of the dynamic platform can be relieved. The more degrees of freedom the dynamic platform has, the longer the flexible legs may be. The more electrical connections the ultrasound transducer has, the more flexible legs may the flexible connection structure have. Advantegously, by automatically reorienting the dynamic platform based on a sensor information, the ultrasound transducer arranged on the dynamic platform can track moving parts inside a body.

By filling the housing of the ultrasound system with a liquid and/or gel, ultrasound energy can be coupled from the ultrasound transducer outside of the housing during reorientation of the ultrasound transducer arranged on the dynamic platform. Advantageously, by embedding the dynamic platform comprising the ultrasound transducer into a solid material of a spherical shape, net displacement of water can be minimized and therefore the dynamic platform can be rapidly reoriented.

By providing the ultrasound transducer arranged on the dynamic platform with an array of transducer elements, field of view of the ultrasound transducer at a respective orientation of the dynamic platform can be further expanded by electronic steering of an ultrasound beam transmitted by collectively operable array of the transducer elements.

By integrating the ultrasound system in a patch and/or wearable device, broad field of view ultrasound images with high resolution can be obtained by repetitive reorientation of the dynamic platform comprising the ultrasound transducer.

### BRIEF DESCRIPTION OF DRAWINGS

These and other features, aspects, and advantages of the apparatus, systems and methods of the present disclosure will become better understood from the following description, appended claims, and accompanying drawing wherein:
FIGs 1A-1B illustrate a reorientable ultrasound system;
FIGs 2A-2B illustrate a flexible connection structure;
FIGs 3A-3B illustrate a housing partially filled with a liquid.

### DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

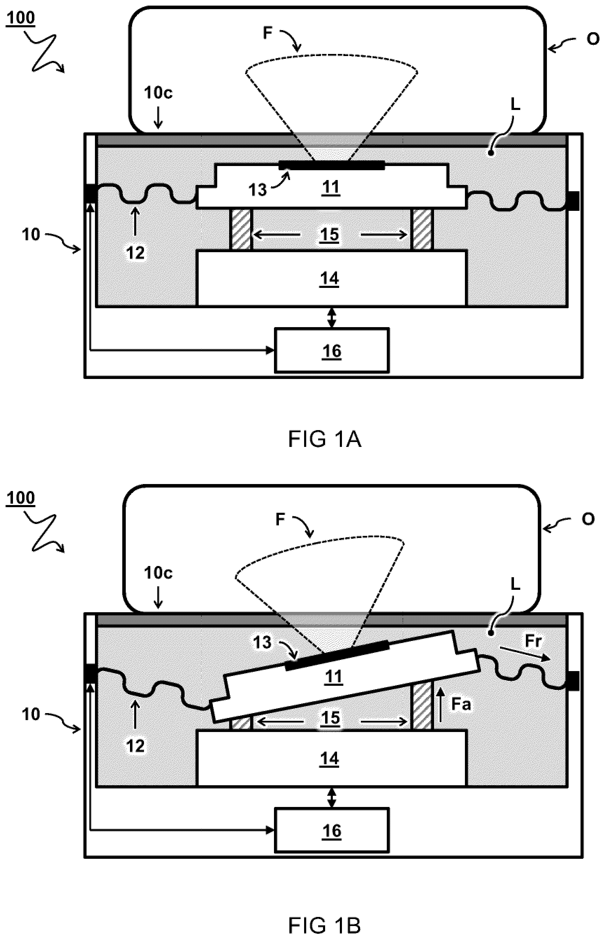

FIGs 1A- 1B illustrate a reorientable ultrasound system comprising a housing 10 with a contact surface 10c for contacting an object O, and a dynamic platform 11 comprising an ultrasound transducer 13. The dynamic platform 11 is suspended within the housing 10 via a flexible connection structure 12. The flexible connection structure 12 is configured to facilitate reorientation of the dynamic platform 11 with respect to the contact surface 10c for modifying a field of view F of the ultrasound transducer 13 on the object O.

In some embodiments, the housing 10 and/or the dynamic platform 11 are formed of an inelastic material, e.g. flexible printed-circuit board (PCB). Alternatively, or in addition, the housing 10 is formed of a material that may bent but doesn't stretch. In one embodiment, stiffness of the housing and/or the dynamic platform 11 is enhanced by adding a stiffener in the PCB design and/or attaching a support to the flexible PCB, e.g. formed of a Polymethyl Methacrylate material.

In some embodiments, the housing 10 comprises a multi-layered PCB with embedded electrical connections and/or electronic components. In yet another or further embodiment, the housing 10 may comprise acoustic window adjacent to the contact surface 10c. Preferably, the thickness of the acoustic window is less than one tenth of the acoustic wavelength emitted by the ultrasound transducer 13. Alternatively, or in addition, the acoustic window comprises a focusing lens for focusing and/or directing ultrasound energy from the ultrasound transducer 13 to the object O. In one embodiment, the ultrasound transducer 13 comprises a (flexible) MEMS, PZT, or polymer transducer. In one embodiment, the ultrasound transducer 13 is configured to emit ultrasound waves at a frequency in a range from one, up to ten, twenty, or more megahertz. In some embodiments, the ultrasound transducer 13 comprises backing absorbing and/or reflecting ultrasound energy emitted in a direction opposite to the contact surface 10c, e.g. rubber-based backing or air-backing. Alternatively, or in addition, the ultrasound transducer 13 comprises no backing. In some embodiments, the ultrasound transducer 13 is wirebonded to an electrical connection fanning out on the dynamic platform 11. In other or further embodiments, the dynamic platform 11 comprises soldered and/or surface mounted electronics, or MEMS components formed by a lithography process. In some embodiments, the ultrasound transducer 13 is powered from a battery arranged in the housing 10. In other or further embodiments, the ultrasound transducer 13 is powered wirelessly. In yet other or further embodiments, the flexible connection structure 12 comprises a flexible PCB and/or flexible substrate, e.g. formed of a Polyethylene Terephthalate material.

In some embodiments, the flexible connection structure 12 comprises electrical connections for electrically connecting the ultrasound transducer 13 disposed on the dynamic platform 11 with a controller 16 arranged in the housing 10. In one embodiment, the electrical connections are arranged on a top or bottom surface of the flexible connection structure 12, or sandwiched there in-between. In another or further embodiment, the electrical connections are configured to transfer analogue and/or digital signals.

In some embodiments, the flexible connection structure 12 is formed of a stretchable material configured to elongate and/or stretch upon applied force Fa caused by reorientation of the dynamic platform 11. In one embodiment, the flexible connection structure 12 is configured to elongate before it breaks by at least a factor of 1.1, 1.2, 1.5 or more. In another or further embodiment, the flexible connection structure 12 is formed of a resilient and/or elastic material, e.g. rubber, configured to exert a restoring force Fr for recovering the field of view F to its initial state when the applied force Fa is removed. In another or further embodiment, the flexible connection structure 12 is made resilient and/or elastic by a patterning process. The patterning process is preferred when the flexible connection structure 12 comprises electrical connections which are inherently not resilient/elastic and may break if disposed on a resilient/elastic material. In one embodiment, the flexible connection structure 12 compries a patterned Polyethylene Terephthalate foil.

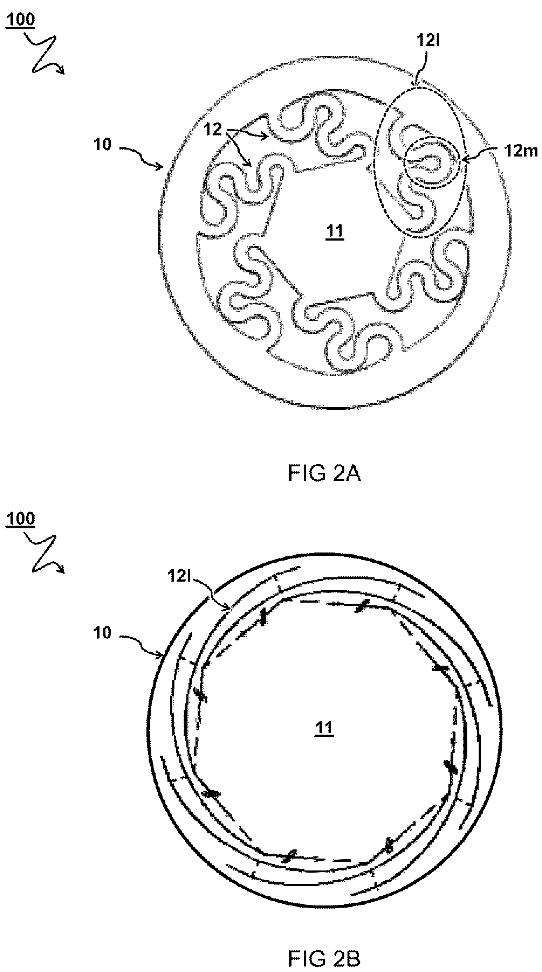

FIGs 2A-2B illustrate a flexible connection structure 12 comprising a plurality of flexible legs 121 with at least one electrical connection (not shown) formed via a respective flexible leg of the plurality of flexible legs 121. In one embodiment, the respective flexible leg comprises a stress-relieving structure configured to relieve stress in the at least one electrical connection by flexing and/or stretching of the respective flexible leg during reorientation of the dynamic platform 11. In some embodiments, the plurality of flexible legs 121 may have various shapes depending on the number of electrical connections to the ultrasound transducer 13 and/or degrees of freedom of the dynamic platform 11. For example, each of the flexible legs may form a meander loop for relieving stress caused by forces induced during reorientation of the dynamic platform 11. In other or further embodiments, the plurality of flexible legs 121 comprises at least three flexible legs arranged between an outer circumference of the dynamic platform 11 and an inner circumference of the housing 10. Preferably, the at least three flexible legs are arranged equidistantly along the circumference of the dynamic platform 11. Alternatively, or in addition, each of the at least three flexible legs is arranged at a respective corner of the dynamic platform 11.

In some embodiments, a controller is configured to control reorientation of the dynamic platform 11 based on a sensor information. In one embodiment, the sensor information comprises ultrasound signal from an object O and the controller is configured to reorient the dynamic platform 11 based on the ultrasound signal, e.g. for tracking moving parts of the object O. Alternatively, or in addition, the sensor information comprises accelerometers, and/or gyroscopes. In other or further embodiments, the ultrasound system 100 comprises an actuator 14 coupled to the controller 16 configured to control orientation of the dynamic platform 11 within the housing 10. In one embodiment, the actuator 14 comprises a micromotor, e.g. a piezoelectric motor, an electromagnetic means, e.g. a solenoid, and/or a pneumatic means, e.g., a micropump. In another or further embodiment, the actuator 14 is coupled to the dynamic platform 11 via a contactless means 15, e.g. electromagnetic field. Alternatively, or in addition, the actuator 14 is coupled to the dynamic platform 11 via pneumatic and/or hydraulic means. In yet another or further embodiment, the actuator 14 comprises a shape memory alloy wire disposed on and/or forming the flexible connection structure 12. In another or further embodiment, the shape memory alloy wire has multiple functionality, e.g. providing an electrical connection and facilitating reorientation of the dynamic platform. Preferably, the shape memory alloy wire comprises a Flexinol wire. Alternatively, or in addition, other shape memory alloy wires can be envisaged, e.g. wires capable to contract by at least two, five, ten, up to twenty percent of more.

In some embodiments, reorientation of the dynamic platform 11 comprises (in-plane) rotation, (out-of-plane) tilting, and/or (in-plane and/or out-of-plane) translation of the dynamic platform 11 with respect to the contact surface 10c. In one embodiment, the (out-of-plane) tilting causes meandering of the flexible connection structure 12, e.g. by thirty, ninety, up to one hundred and twenty degrees. In another or further embodiment, the (in-plane) rotation causes spiraling of the flexible connection structure 12, e.g. by thirty, ninety, up to one hundred and twenty degrees.

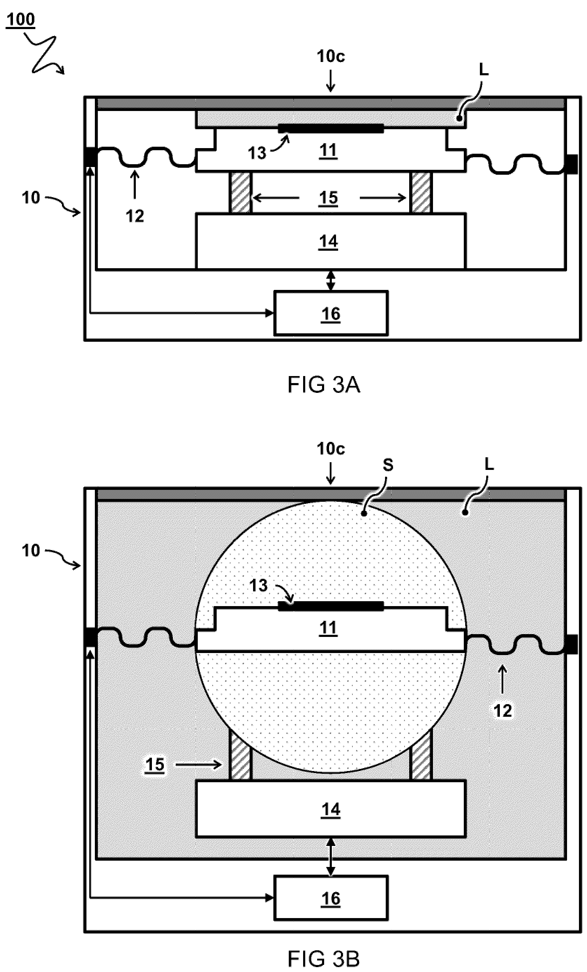

FIGs 3A-3B illustrate a housing 10 filled, at least between the ultrasound transducer 13 and the contact surface 10c, with a liquid material L for coupling ultrasound energy from the ultrasound transducer 13 inside the housing 10 through the contact surface 10c to an object outside of the housing 10. For example, the liquid may comprises water and/or ethylene glycol. In one embodiment, the ultrasound transducer 13 and the dynamic platform 11 are embedded in a solid material S for minimizing movement of the liquid material L during rotation of the dynamic platform 11. For example, the solid material may comprise hydrogel, water and/or ethylene glycol disposed in balloon.

In some embodiments, the ultrasound transducer 13 comprises an array of transducer elements, each transducer configured to transduce ultrasound waves, individually and/or collectively operable for electronic steering, focusing, and/or tracking of structures inside an object O with an ultrasound beam transmitted by the array of transducer elements. In one embodiment, the ultrasound transducer 13 is configured to collect ultrasound data at a plurality reorientations of the dynamic platform 11 for broadening view in an ultrasound image by stitching the ultrasound data. In another or further embodiment, the ultrasound transducer 13 is configured to collect ultrasound data at a plurality reorientations of the dynamic platform 11 for increasing resolution of an ultrasound image by combining the ultrasound data. In some embodiments, the view broadening and/or the resolution increasing in the ultrasound image comprises interpolation of the ultrasound data. In yet other or further embodiments, the ultrasound system 100 is integrated in a patch and/or a wearable device for continous and/or long term monitoring of body parts or structures at a plurality of orientations of the dynamic platform 11.

In other or further embodiments, the ultrasound system 100 is used in a method of measuring an object O. In one embodiment, the method comprises contacting the object O by a contact surface 10c of a housing 10 of an ultrasound system 100 and using an ultrasound transducer 13 of the ultrasound system 100 to measure the object O. In another or further embodiment, the ultrasound transducer 13 is formed as part of a dynamic platform 11 suspended inside the housing 10. In some embodiments, the dynamic platform 11 is connected to the housing 10 via a flexible connection structure 12. In another or further embodiment, the method comprises reorienting the dynamic platform 11 inside the housing 10 with respect to the contact surface 10c, while the dynamic platform 11 is held suspended via the flexible connection structure 12, for modifying a field of view F of the ultrasound transducer 13 on the object O.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise. Where one claim refers to another claim, this may indicate synergetic advantage achieved by the combination of their respective features. But the mere fact that certain measures are recited in mutually different claims does not indicate that a combination of these measures cannot also be used to advantage. The present embodiments may thus include all working combinations of the claims wherein each claim can in principle refer to any preceding claim unless clearly excluded by context.

## Claims

1. An ultrasound system (100) comprising
a housing (10) comprising a contact surface (10c) for contacting an object (O);
a dynamic platform (11) comprising an ultrasound transducer (13), wherein the dynamic platform (11) is suspended inside the housing (10), wherein the dynamic platform (11) is connected to the housing (10) via a flexible connection structure (12);
wherein the flexible connection structure (12) is configured to allow reorientation of the dynamic platform (11) inside the housing (10) with respect to the contact surface (10c) for modifying a field of view (F) of the ultrasound transducer (13) on the object (O).

2. The ultrasound system (100) according to the preceding claim comprising an actuator (14) configured to actuate the dynamic platform (11) for reorienting the dynamic platform (11) with respect to the contact surface (10c).

3. The ultrasound system (100) according to any of the preceding claims, wherein, the actuator (14) comprises a flexible and/or contactless coupling to the dynamic platform (11).

4. The ultrasound system (100) according to the preceding claim, wherein the actuator (14) comprises at least one shape memory alloy wire forming part of the flexible connection structure (12).

5. The ultrasound system (100) according to any of the preceding claims, wherein the flexible connection structure (12) comprises of a stretchable resilient material or structure configured to elongate and/or stretch upon a force (Fa) being applied by the actuator (14) on the dynamic platform (11), and exert a restoring force (Fr) on the dynamic platform (11) for recovering a default orientation of the dynamic platform (11) when the applied force (Fa) is removed.

6. The ultrasound system (100) according to claim 2 or any other claim dependent thereon, wherein the actuator (14) is configured to reorient the dynamic platform (11) by at least one of rotating, tilting, and translating of the dynamic platform (11) inside the housing (10) with respect to the contact surface (10c).

7. The ultrasound system (100) according to any of the preceding claims comprising a controller (16) configured to control reorientation of the dynamic platform (11) based, at least in part, on measurements of the ultrasound transducer (13).

8. The ultrasound system (100) according to any of the preceding claims, wherein the flexible connection structure (12) incorporates a set of electrical connections for electrically connecting the ultrasound transducer (13) disposed on the dynamic platform (11).

9. The ultrasound system (100) according to any of the preceding claims, wherein the flexible connection structure (12) comprises a plurality of flexible legs (121) wherein at least one electrical connection is formed via a respective flexible leg of the plurality of flexible legs (121), wherein the respective flexible leg comprises a stress-relieving structure configured to relieve stress in the at least one electrical connection by flexing and/or stretching of the respective flexible leg during reorientation of the dynamic platform (11).

10. The ultrasound system (100) according to any of the preceding claims, wherein the housing (10) is filled, at least between the ultrasound transducer (13) and the contact surface (10c), with a liquid material (L) for coupling ultrasound energy from the ultrasound transducer (13) inside the housing (10), via the liquid material (L), through the contact surface (10c) to the object (O) outside of the housing (10).

11. The ultrasound system (100) according to the preceding claim, wherein the dynamic platform (11) comprising the ultrasound transducer (13) is embedded in a sphere of solid material (S) suspended in the liquid material (L).

12. The ultrasound system (100) according to any of the preceding claims, wherein the ultrasound transducer (13) comprises an array of transducer elements, each transducer configured to transduce ultrasound waves, individually and/or collectively operable, at each of a plurality of orientations of the dynamic platform (11).

13. The ultrasound system (100) according to any of the preceding claims, wherein the ultrasound transducer (13) is configured to collect ultrasound data at a plurality orientations of the dynamic platform (11) and combine the collected ultrasound data to broaden a view in an ultrasound image by stitching the ultrasound data and/or increase a resolution of an ultrasound image.

14. The ultrasound system (100) according to any of the preceding claims, wherein the ultrasound system (100) is integrated in a patch and/or a wearable device.

15. A method of measuring an object (O), the method comprising
contacting the object (O) by a contact surface (10c) of a housing (10) of an ultrasound system (100);
using an ultrasound transducer (13) of the ultrasound system (100) to measure the object (O), wherein the ultrasound transducer (13) is formed as part of a dynamic platform (11) suspended inside the housing (10), wherein the dynamic platform (11) is connected to the housing (10) via a flexible connection structure (12); and
reorienting the dynamic platform (11) inside the housing (10) with respect to the contact surface (10c), while the dynamic platform (11) is held suspended via the flexible connection structure (12), for modifying a field of view (F) of the ultrasound transducer (13) on the object (O).
